(19) **Europäisches Patentamt / European Patent Office / Office européen des brevets**

(11) **EP 3 773 514 B1**

(12) **EUROPEAN PATENT SPECIFICATION**

(45) Date of publication and mention of the grant of the patent:
**04.05.2022 Bulletin 2022/18**

(21) Application number: **19722513.9**

(22) Date of filing: **10.04.2019**

(51) International Patent Classification (IPC):
**A61K 9/48** *(2006.01)*     **A61K 31/05** *(2006.01)*
**A61K 31/366** *(2006.01)*     **A61K 31/7024** *(2006.01)*
**A61K 36/63** *(2006.01)*     **A61K 36/88** *(2006.01)*
**A61K 36/899** *(2006.01)*

(52) Cooperative Patent Classification (CPC):
(C-Sets available)
**A61K 9/4875; A61K 9/4816; A61K 9/4825; A61K 9/4858; A61K 31/05; A61K 31/366; A61K 31/7024; A61K 36/63; A61K 36/88; A61K 36/899**     (Cont.)

(86) International application number:
**PCT/EP2019/059117**

(87) International publication number:
**WO 2019/197481 (17.10.2019 Gazette 2019/42)**

(54) **COMPOSITION, IN THE FORM OF A SOFT CAPSULE, COMPRISING A COMBINATION OF EXTRACTS FROM OLIVE FRUIT, RED YEAST RICE AND CROCUS SATIVUS L. AND METHOD OF PREPARING THE SAME**

ZUSAMMENSETZUNG IN FORM EINER WEICHEN KAPSEL MIT EINER KOMBINATION VON EXTRAKTEN AUS OLIVENFRÜCHTEN, ROTEM HEFEREIS UND CROCUS SATIVUS L. UND VERFAHREN ZUR HERSTELLUNG DAVON

COMPOSITION, SOUS LA FORME D'UNE CAPSULE MOLLE, COMPRENANT UNE COMBINAISON D'EXTRAITS D'OLIVE, RIZ ROUGE DE LEVURE ET DE CROCUS SATIVUS L. ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.04.2018 EP 18167383**
**22.03.2019 EP 19386018**

(43) Date of publication of application:
**17.02.2021 Bulletin 2021/07**

(73) Proprietor: **Tseti, Ioulia**
**145 61 Kifissia Attikis (GR)**

(72) Inventor: **Tseti, Ioulia**
**145 61 Kifissia Attikis (GR)**

(74) Representative: **Wibbelmann, Jobst**
**Wuesthoff & Wuesthoff**
**Patentanwälte PartG mbB**
**Schweigerstrasse 2**
**81541 München (DE)**

(56) References cited:
**WO-A2-2009/013596**

• **Gabriele Reich: "Formulation and physical properties of soft capsules" In: Anonymous: "Pharmaceutical Capsules", 1 May 2004 (2004-05-01), Pharmaceutical Press, XP002784475, page 201-212, page 205, right-hand column, paragraph 4 page 205, left-hand column, lines 1-2 page 205, right-hand column, paragraph 1**

- Anonymous: "Modern Pharmaceutics, volume 1, basic principles and systems", 2010, CRC Press, XP002784476, page 410-411, page 411, paragraph 2-3
- TSHONGO MUHINDO CHRISTIAN ET AL: "Efficacy and safety of a combination of red yeast rice and olive extract in hypercholesterolemic patients with and without statin-associated myalgia", COMPLEMENTARY THERAPIES IN MEDICINE, vol. 35, 9 November 2017 (2017-11-09), pages 140-144, XP085265272, ISSN: 0965-2299, DOI: 10.1016/J.CTIM.2017.10.014
- ENNIAN LENG ET AL: "Synergistic effect of phytochemicals on cholesterol metabolism and lipid accumulation in HepG2 cells", BMC COMPLEMENTARY AND ALTERNATIVE MEDICINE, BIOMED CENTRAL LTD, LONDON, UK, vol. 18, no. 1, 5 April 2018 (2018-04-05), pages 1-10, XP021255173, DOI: 10.1186/S12906-018-2189-6

Remarks:

The file contains technical information submitted after the application was filed and not included in this specification

(52) Cooperative Patent Classification (CPC): (Cont.)

C-Sets
A61K 31/05;
A61K 31/366;
A61K 31/7024;
A61K 36/63;
A61K 36/88;
A61K 36/899

Remarks:

The file contains technical information submitted after the application was filed and not included in this specification

## Description

**Field of the Invention**

The present invention relates to a stable non-aqueous composition comprising the unique combination of three water-soluble extracts from olive fruit, Red Yeast Rice and *Crocus sativus L.* stigmas and a method for preparing the same. This composition is suitable for encapsulation in soft capsules and is standardized in certain quantities of hydroxytyrosol, monacolin K and crocin, which act synergistically for the protection of blood lipids from oxidative stress.

**Background of the Invention**

Hydroxytyrosol (HT) is a phenolic compound naturally occurring in olive fruit and olive oil, and it was first discovered and extracted from the wastewater that resulted when green olives were rinsed in the process of making olive oil. Several studies have revealed the antioxidative and antidiabetic effect of olive oil polyphenols, including hydroxytyrosol. Recent studies on rats, following a model of diet-induced metabolic syndrome, high in carbohydrates and fat, showed that treatment with hydroxytyrosol decreased adiposity, improved impaired glucose and insulin tolerance and improved endothelial function with lower systolic blood pressure.

According to the Commission Regulation (EU) No 432/2012 on establishing a list of permitted health claims made on foods, olive oil polyphenols contribute to the protection of blood lipids from oxidative stress with a daily intake of at least 5 mg of hydroxytyrosol and its derivatives (e.g. oleuropein complex and tyrosol). These amounts, if provided by moderate amounts of olive oil, can be consumed in the context of a balanced diet (e.g. 20 g olive oil per day). However, taking into account that the concentrations in olive oils are not consistent, the consumption of this amount of polyphenols is not standardized. Therefore, a composition providing regulated amounts of hydroxytyrosol on a daily basis is necessary.

Red Yeast Rice is the product of rice fermented with Monascus purpureus yeast. The active ingredient in Red Yeast Rice is Monacolin K. Monacolin K belongs in the family of statins and presents similar properties as the known statin Lovastatin in the regulation of blood cholesterol levels. More specifically it inhibits hydroxylmethyl glutaryl-CoA (HMG-CoA) reductase in liver from producing cholesterol, thus lowering its level in blood. According to the list of permitted health claims made on foods, a daily intake of 10 mg of monacolin K from Red Yeast Rise contributes to the maintenance of blood cholesterol levels. Commercial products of Red Yeast Rice are freely soluble in water and present variable contents of monacolin K, from 0.5% to more than 10%.

*Crocus sativus L.* (saffron) is a perennial spicy herb of Iridaceae family and is characterized by its strong aroma, its bitter taste and its orange-reddish pigment. These characteristics are due to more than 150 volatile (mainly terpenes) and nonvolatile (mainly carotenoids) active components, and primarily due to four major bioactive compounds, namely, safranal, picrocrocin, crocetin and crocin. Saffron is regarded as tonic and antidepressant and has been used in various ancient cultures for strengthening digestion, relieving coughs, smoothing menstruation, relaxing muscle spasms, calming anxiety and improving mood. The stigmas of spice saffron are increasingly attracting attention because of their much functionality, such as in metabolic disorders, cardiovascular diseases, depression, anxiety and insomnia. Clinical studies in rats revealed the hypolipidemic effect of crocin on pancreatic lipase activity, rendering it a suitable candidate for use in a hypolipidemic treatment.

EP2170090 relates to nutritional and dietary products to be used as a source of hydroxytyrosol for preventing or treating cardiovascular diseases, plaque build-up in the arteries, arterial hypertension, and metabolic syndrome. The invention also relates to a process for preparing fortified oil-containing products with olive extract, i.e. for preparing so-called functional food. The process consists of mixing an aqueous solution of olive extract with olive oil in the presence of an emulsifier. Then, the mixture left to rest for at least 72 hours and the aqueous phase is decanted in order to obtain the fortified enriched oil. Furthermore the above-mentioned emulsion of olive extract in liquid form and edible oil was used to prepare soft gel capsules. The aforesaid method of enriching the edible oil with hydroxytyrosol is time-consuming due to the separation step of the aqueous from the oily phase.

WO2010/069951 relates to pharmaceutical formulations for oral administration comprising omega-3 and omega-6 fatty acids and natural or semi-synthetic statins. The two active ingredients are kept isolated from each other inside the same dosage unit. The fatty acids are encapsulated in soft gelatin capsules, which are further nebulized with a suspension containing statins and other active ingredients. According to invention embodiments, the final product contains 500 mg of omega-3 fatty acids inside each capsule and 200 mg of fermented red rice, corresponding to 3 mg of statins, on the capsule coating.

WO2012114133 relates to a process for the enrichment of olive oil with biologically active substances obtained by the extraction of powder and stigmas of plant Saffron (*Crocus sativus L.*) in alcohol or water. All enriched solutions present higher antioxidant capacity than the originals, thus revealing the synergistic effect of the combination active ingredients coming from dried extracts of *Crocus sativus L.* and olive oil. However, the content of the active ingredients (crocin, crocetin, safranal) in the *Crocus sativus L.* extract and therefore in the enriched olive oil is not specified.

A stable non-aqueous composition consisting of the unique combination of three water-soluble extracts, namely, Olive fruit extract, Red Yeast Rice extract and *Crocus sativus L.* extract, standardized in hydroxytyrosol, monacolin K and crocin, respectively, all dispersed in an edible vegetable oil, is not reported in the prior art.

The present invention is based on the discovery that these three water-soluble extracts provide a synergistic effect in terms of the antioxidant efficacy of the active ingredients comprised in the extracts.

A stable non-aqueous composition suitable for inclusion in a soft capsule should, however, meet certain criteria. Thus, it is important that such composition is stable and provides a homogenous distribution of reactive ingredients in, for instance, soft capsules. The applicant has undertaken significant efforts to this end. Nevertheless, following conventional wisdom, it appeared difficult to avoid the formation of large aggregates in the suspension of the active ingredients in an edible oil (see experimental section of this application).

WO 2009/013596 A2 discloses the use of soft capsule compositions comprising hydroxytyrosol in a hydrophobic matrix (olive oil) for the prevention and treatment of cardiovascular diseases.

## Summary of the Invention

Accordingly, a first object of the invention relates to a nutritional product in the form of a soft capsule, suitable for oral administration, which provides the unique combination of the active ingredients hydroxytyrosol, monacolin K and crocin. The composition of this invention combines the unique properties of three components, which can act synergistically in the maintenance of blood cholesterol levels in a subject, by protecting blood lipids from oxidative stress.

A second object of the invention relates to a non-conventional but feasible method of preparing a non-aqueous homogeneous composition comprising of hydroxytyrosol, monacolin K and crocin. In this context, the invention solves the problem of keeping the active ingredients isolated from each other in the same preparation.

## Description of the invention

The invention relates to a nutritional product in the form of a soft capsule, suitable for oral administration, comprising as active ingredients the combination of olive fruit extract standardized to at least 7% of hydroxytyrosol, Red Yeast Rice extract standardized to at least 4% of monacolin K and *Crocus sativus L.* extract standardized to at least 25% of crocin, wherein the combination is encapsulated in the soft capsule and homogeneously distributed in a hydrophobic matrix.

In the present invention, hydroxytyrosol is in the form of an olive fruit extract standardized to at least 7% of hydroxytyrosol. Such extracts are commercially available and come from the extraction of olive fruits and olive fruit waste waters under known methods. The assay of hydroxytyrosol in a standardized olive fruit extract is evaluated with the aid of high-performance liquid chromatography (HPLC) at 280 nm.

In the present invention, monacolin K is in the form of Red Yeast Rice extract standardized to at least 4% of monacolin K. Water-soluble Red Yeast Rice extracts containing up to 25% monacolin K are known in the prior art and are commercially available. The assay of monacolin K in a standardized Red Yeast Rice extract is evaluated with the aid of high-performance liquid chromatography (HPLC) at 237 nm.

In the present invention, crocin is in the form of *Crocus sativus L.* (saffron) extract standardized to at least 25% of crocin. GR1008908 refers to a process of making stable water-soluble extracts from the stigmas of *Crocus sativus L.,* rich in crocin and suitable for prevention of atherosclerosis.

The assay of crocin in a standardized extract from the stigmas of *Crocus sativus L.* is evaluated with the aid of high-performance liquid chromatography (HPLC) at 440 nm.

In a preferred embodiment the dosage unit relates to a soft capsule having a weight between 600 and 1200 mg, said capsule contains a stable non-aqueous composition comprising of: 5-8 % w/w of an olive fruit extract standardized to at least 7 % of hydroxytyrosol, 10-30% w/w of Red Yeast Rice extract standardized to at least 3% monacolin K, 10-20% w/w *Crocus sativus L.* dried extract standardized to at least 25% of crocin, 25-50% w/w edible oil and a suitable emulsifier.

In a preferred embodiment, the quantity of olive fruit extract, standardized in 7% hydroxytyrosol, is in the range of 30 to 70 mg per dosage unit, which corresponds from 2 to 5 mg of pure hydroxytyrosol per dosage unit.

In a preferred embodiment, the quantity of the standardized in 7% hydroxytyrosol olive fruit extract is 35 mg per dosage unit, which corresponds to 2.5 mg of pure hydroxytyrosol per dosage unit.

In a preferred embodiment, the quantity of Red Yeast Rice extract, standardized in 4% monacolin K, is in the range of 50 to 200 mg per dosage unit, which corresponds from 2 to 8 mg of monacolin K per dosage unit.

In a preferred embodiment, the quantity of Red Yeast Rice extract, standardized in 4% monacolin K, is 125 mg per dosage unit, which corresponds to 5 mg of monacolin K per dosage unit.

In a preferred embodiment, the quantity of *Crocus sativus L.* extract, standardized in 25% crocin, is in the range of 70 to 200 mg per dosage unit, which corresponds from 17.5 to 50 mg of crocin per dosage unit.

In a preferred embodiment, the quantity of *Crocus sativus L.* extract, standardized in 25% crocin, is 100 mg per dosage unit, which corresponds to 25 mg of crocin per dosage unit.

In a preferred embodiment, olive fruit extract, Red Yeast Rice extract and *Crocus sativus L.* extract are dispersed in an edible vegetable oil, selected from coconut oil, corn oil, cotton seed oil, olive oil, palm oil, peanut oil, rape seed oil, safflower oil, sesame oil, soy bean oil, and sunflower oil.

In a preferred embodiment, the dispersion of olive fruit extract, Red Yeast Rice extract and *Crocus sativus L.* extract takes place in an edible vegetable oil, the quantity of which is in the range from 200 mg to 400 mg per dosage unit, more preferably is 240 mg per dosage unit.

In a preferred embodiment, olive fruit extract, Red Yeast Rice extract and *Crocus sativus L.* extract are dispersed in olive oil, which is also rich in polyphenols, and acts synergistically with the above-mentioned three extracts in the protection of blood lipids from oxidative stress.

In a preferred embodiment, the composition further comprising of a suitable emulsifier, preferably lecithin, the quantity of which is in the range from 80 mg to 160 mg per dosage unit, more preferably is 100 mg per dosage unit.

In a preferred embodiment the dosage unit is in the form of a soft capsule, each capsule comprising of the ingredients of the capsule shell and encapsulates the composition of the invention comprising of 2.5 mg hydroxytyrosol, 5 mg monacolin K, 25 mg crocin, 240 gr of an edible oil and 100 gr of lecithin.

In a preferred embodiment the dosage unit is in the form of a soft capsule, each capsule comprising of the ingredients of the capsule shell and encapsulates the composition of the invention comprising of 35 mg olive fruit extract, 125 mg Red Yeast Rice extract and 100 mg *Crocus sativus L.* extract, 240 gr of an edible oil and 100 gr of lecithin.

The invention also relates to a method of preparing a stable non-aqueous composition comprising the unique combination of a dried olive fruit extract, a Red Yeast Rice extract and a *Crocus sativus L.* dried extract presenting a synergistic effect in the regulation of blood cholesterol levels in a subject.

It was proved difficult to prepare a homogeneous dispersion following common procedures from the prior art i.e. by preparing a blend of the three water-soluble solid extracts and subsequently dispersing the solid blend into a hydrophobic matrix with the aid of stirring or agitation and optionally with the aid of heating. Experiments carried out following a well-established procedure, where dried extracts of olive fruit, Red Yeast Rice and *Crocus sativus L.* are mixed together and the mixture is blended using a drum mixer of 50 L capacity, at 7 rpm rotating speed for 120 min. Blend uniformity tests performed on 3 samples from different positions of the drum mixer. Major uniformity deviations observed in the final blend, especially in terms of hydroxytyrosol assay even after 240 min of blending time (Tables 1 and 2).

Table 1: Uniformity results of the solid mixture of the three extracts after blending for 120 min

| Ingredient | Quantity in final blend | Assay in active Ingredient | | |
|---|---|---|---|---|
| | | Sample 1 | Sample 2 | Sample 3 |
| Olive fruit extract (hydroxytyrosol) | 3.5 Kg | 86.1 % | 106.8 % | 90.2 % |
| Red Yeast Rice extract (monacolin K) | 12.5 Kg | 101.2 % | 99.6 % | 96.5 % |
| *Crocus sativus L.* extract (Crocin) | 10.0 Kg | 102.0 % | 96.3 % | 99.7% |

Table 2: Uniformity results of the solid mixture of the three extracts after 240 min blending

| Ingredient | Quantity in final blend | Assay in active Ingredient | | |
|---|---|---|---|---|
| | | Sample 1 | Sample 2 | Sample 3 |
| Olive fruit extract (hydroxytyrosol) | 3.5 Kg | 108.6 % | 93.8 % | 104.8 % |
| Red Yeast Rice extract (monacolin K) | 12.5 Kg | 97.8. % | 104.4 % | 100.6 % |
| *Crocus sativus L.* extract (Crocin) | 10.0 Kg | 99.5 % | 97.3 % | 96.6% |

It was supposed that these deviations would decrease during the dispersion of the blend into the edible oil under agitation with the aid of heating and in the presence of an emulsifier. In this context, 24 Kg of edible oil mixed with 10 kg of lecithin in an enameled reactor at 600 rpm and at 60 °C. The blend of the three extracts gradually added in the above oily suspension and the whole mixed under the same conditions. However, large aggregates formed in the oily suspension, which were not homogenized even after stirring for 120 min and after passing through a colloidal mill (300 Lt/h).

Surprisingly, it was found that a non-aqueous, oily, homogeneous and stable dispersion consisting of the extracts from olive fruits, Red Yeast Rice and *Crocus sativus L.* obtained by following the next steps:

a) Each of the dried extracts was separately sieved through a 60 mesh sieve to obtain a particle size of less than 250 μm.

b) the sieved olive fruit extract is mixed with an edible oil and an emulsifier, at a temperature in the range of 45 °C and 75 °C.

c) the sieved Red Yeast Rice extract is dispersed in the oily mixture of the second step, following by stirring at the same temperature for a time period between 1 and 3 hours.

d) the oily mixture of the third step is cooled down to a temperature in the range between 25 °C and 35 °C and then homogenized by passing through a colloidal mill (300 Lt/h).

e) the sieved *Crocus sativus L.* dried extract is dispersed in the homogenous oily dispersion of the third step, following by stirring at a temperature between 25 °C and 35 °C for a time period between 1 and 3 hours.

f) the final oily non-aqueous mixture is cooled down to room temperature, more preferably at 25 °C and further homogenized by passing through a colloidal mill (300 Lt/h).

Uniformity tests performed in order to evidence that the final oily non-aqueous composition is homogeneous. Table 3 presents the quantity of the active ingredient of each extract in the blend and its assay in each sample.

Table 3: Uniformity results of the oily non-aqueous composition as obtained following the steps a) to f).

| Ingredient | Quantity in final blend | Assay in active Ingredient | | |
|---|---|---|---|---|
| | | Sample 1 | Sample 2 | Sample 3 |
| Olive fruit extract (hydroxytyrosol) | 3.5 Kg | 99.8 % | 100.6 % | 99.2 % |
| Red Yeast Rice extract (monacolin K) | 12.5 Kg | 100.2 % | 99.6 % | 99.5 % |
| *Crocus sativus L.* extract (Crocin) | 10.0 Kg | 99.3 % | 100.9 % | 99.7% |

Uniformity tests summarized in Table 3 show that the composition obtained following the steps a) to f) is homogeneous and fully retains the percentages of the active ingredients, namely, hydroxytyrosol, monacolin K and crocin, as those evaluated by high-pressure liquid chromatography (HPLC). The composition of the invention remain stable after 3 and 6 months storage at normal and accelerated conditions of temperature and relative humidity.

Table 4. Stability studies of the oily non-aqueous composition of the invention after storage at normal and accelerated conditions.

| Ingredient | Assay in active Ingredient | | | |
|---|---|---|---|---|
| | After 3 months storage at 25°C and 40% RH | After 3 months storage at 40°C and 75% RH | After 6 months storage at 25°C and 40% RH | After 6 months storage at 40°C and 75% RH |
| Olive fruit extract (hydroxytyrosol) | 99.5% | 98.9% | 98.4% | 97.1% |
| Red Yeast Rice extract (monacolin K) | 99.9% | 99.0% | 98.1% | 96.8% |
| *Crocus sativus L.* extract (Crocin) | 99.0% | 97.6% | 97.7% | 95.9% |

The stable non-aqueous composition of the invention, comprising the unique combination of dried solid extracts from olive fruits, Red Yeast Rice and *Crocus sativus L.* stigmas, is suitable for encapsulation in soft capsules, and can be consumed as a dietary supplement for oral administration.

In a preferred embodiment, the soft capsule shell is manufactured from gelatin, a plasticizer, preferably glycerin and purified water according to known in the state of the art methods. The process for producing gelatin comprise of mixing the components at a high temperature, preferably between 60 °C and 80 °C, under vacuum, for a time period between 3 and 4 hours, preferably 4 hours.

In a preferred embodiment, the soft capsule shell is manufactured from non-animal ingredients including water-soluble polysaccharides, cellulose, micro-crystalline starch derivatives and/or carrageenan extracts, according to known methods. The as prepared soft capsules are intended for use by subjects following a vegetarian diet.

The soft capsule of gelatin or non-gelatin origin can be shaped in various forms, optionally, oval, or oblong or spherical.

## Examples

In a preferred embodiment, the edible olive oil is heated to 50 °C and then the emulsifier is added in portions under agitation. The sieved through a 60 mesh sieve olive fruit extract standardized in hydroxytyrosol is dispersed in the oily mass gradually under stirring, preferably at 600 rpm, maintaining the same temperature. Subsequently, the sieved through a 60 mesh sieve Red Yeast Rice extract is gradually added and the oily mixture is stirred for 120 minutes keeping the temperature at 50 °C. The oily mixture is then cooled to 30 °C and passes through a colloidal mill (300 Lt/h). The sieved through a 60 mesh sieve extract of *Crocus sativus L.* is then added to the homogenous mixture and the oily mixture is stirred at 600 rpm for an additional 2 hours at 25 °C. The final mixture, which consists of the filling mass of the soft capsules, passes through a colloidal mill (300 Lt/h) for optimal homogenization. The mixture is then encapsulated in soft gelatin capsules of oval shape, which subsequently dried in rotating bins with air flow at 20 °C in a low humidity atmosphere, preferably 25% RH, and simultaneous mechanical cleaning of the capsules from the lubricating oil (paraffin oil) with lint-free towels for about 24 hours.

The final composition and the capsule shell components are briefly described in the following table:

| Ingredients | Quantity (mg) per dosage unit |
|---|---|
| *Crocus sativus L.* Extract (25% in Crocin) | 100 |
| Olive fruit extract (7% in hydroxytyrosol) | 35 |
| Red Yeast Rice Extract (4% in Monacolin K) | 125 |
| Edible olive oil | 240 |
| Lecithin | 100 |
| **Ingredients of soft capsule shells** | |
| Gelatin Pharma 195 bloom | 132 |
| Glycerin | 48 |
| Purified water | 20 |

In a preferred embodiment, the edible olive oil is heated to 50 °C and then the emulsifier is added in portions under agitation. The sieved through a 60 mesh sieve olive fruit extract standardized in hydroxytyrosol is dispersed in the oily mass gradually under stirring, preferably at 600 rpm, maintaining the same temperature. Subsequently, the sieved through a 60 mesh sieve Red Yeast Rice extract is gradually added and the oily mixture is stirred for 120 minutes keeping the temperature at 50 °C. The oily mixture is then cooled to 30 °C and passes through a colloidal mill (300 Lt/h). The sieved through a 60 mesh sieve extract of *Crocus sativus L.* is then added to the homogenous mixture and the oily mixture is stirred at 600 rpm for an additional 2 hours at 25 °C. The final mixture, which consists of the filling mass of the soft capsules, passes through a colloidal mill (300 Lt/h) for optimal homogenization. The mixture is then encapsulated in soft non-gelatin capsules of spherical shape, which subsequently dried in rotating bins with air flow at 20 °C in a low humidity atmosphere, preferably 25% RH, and simultaneous mechanical cleaning of the capsules from the lubricating oil (paraffin oil) with lint-free towels for about 24 hours.

The final composition and the capsule shell components are briefly described in the following table:

| Ingredients | Quantity (mg) per dosage unit |
|---|---|
| *Crocus sativus L.* Extract (25% in Crocin) | 100 |
| Olive fruit extract (7% in hydroxytyrosol) | 35 |
| Red Yeast Rice Extract (4% in Monacolin K) | 125 |
| Edible olive oil | 240 |
| Lecithin | 100 |

(continued)

**Ingredients of soft capsule shells**

| | |
|---|---|
| Microcrystalline Starch | 46 |
| Iota-carrageenan | 20 |
| Glycerin | 46 |
| Disodium Phosphate | 2 |
| Purified water | 86 |

The composition of the invention provides a regulated delivery of the three active ingredients, namely, hydroxytyrosol, monacolin K and crocin to a subject, through a standardized dosage unit on a daily base. Each dosage unit, in the form of a single soft capsule, contains 2.5 mg of hydroxytyrosol in the form of olive fruit extract, 5 mg of monacolin K in the form of Red Yeast Rice and 25 mg of crocin in the form of a *Crocus sativus L.* extract. The daily consumption of two soft capsules of Examples 1 and / or 2 provides the claimed amounts of hydroxytyrosol and monacolin K, according to Commission Regulation (EU) No 432/2012 and further provides an enriched to crocin extract, all present a beneficial effect on the maintenance of blood cholesterol levels.

It is within the ability of a skilled person to adjust the quantities of the extracts and prepare compositions according to the invention comprising of different strengths of the active ingredients.

**Antiradical scavenging activity tests.**

The model of scavenging stable radical 2,2-diphenyl-1-picrylhydrazyl radical (DPPH) is a widely used method to evaluate antioxidant capacities of natural products, and it has been used for olive oil and other vegetable oils, as well as for individual antioxidants (Brand-Williams, W. et al. Lebensm.-Wiss. u.-Technol. 1995, 28, 25-30). The spectrophotometric method for assessing the total antioxidant activity is based on the absorbance decrease monitoring of the DPPH· radical in the presence of antioxidants. DPPH· is characterized as a stable free radical due to the delocalization of the spare electron over the molecule. Thus, the molecule cannot dimerize, as would happen with other free radicals. The delocalization gives rise to a deep violet color characterized by an absorption band at about 515 nm. When a DPPH· solution is mixed with a substance which can donate a hydrogen atom, the reduced form is generated, accompanied by the loss of the violet color.

The DPPH radical scavenging activity of the three active components, namely hydroxytyrosol from olive fruit extract, monacolin K from red yeast rice extract and crocins from *Crocus sativus L.* extract and their combination in a composition suitable for oral administration, is measured, in order to reveal the synergism for the three components.

**Experimental Method for DPPH radical scavenging test.**

A solution of DPPH $10^{-3}$ M was prepared by dissolving 0.0394 g of the radical DPPH in 100 mL methanol. The volumetric flask was wrapped in foil and stirred in a vortex apparatus. The solution obtained had a deep purple color and was left in the refrigerator for 2 h in order for the absorbance to be stabilized. A calibration curve of concentration versus absorbance of DPPH was prepared as follows: The $10^{-3}$ M solution of DPPH was diluted with the addition of methanol to prepare DPPH solutions in the range of 5 to 1000 μM ($10^{-4}$ M, $5 \times 10^{-5}$ M, $10^{-5}$ M, and $5 \times 10^{-6}$ M). These solutions were vortexed, left in darkness and their absorbance was measured in a UV-Vis spectrophotometer (Varian Cary 300) at $\lambda_{max}$ = 515 nm and 630 nm.

The calibration curve of absorbance (*Abs*) versus concentration (*Conc*) of DPPH is expressed by the following equations:

$$\lambda = 515 \text{ nm}: Abs = 0.00557*Conc - 0.00070;\ R = 0.99994$$

$$\lambda = 630 \text{ nm}: Abs = 0.00192*Conc - 0.00298;\ R = 0.99997$$

For the antioxidant test, all extracts/compositions tested were dissolved in methanol at the corresponding concentrations (please refer in Tables 1 and 2). Briefly, stock solutions of all extracts were prepared in methanol as following: olive fruit extract 3.60 mg/mL, red yeast rice extract 11.60 mg/mL, *Crocus sativus L.* extract 10.52 mg/mL. The final solutions resulted from dilutions of the stock solutions. Olive fruit extract solution prepared with 1 mL of corresponding stock solution diluted to 5 mL with methanol, red yeast rice extract solution prepared with 2 mL of corresponding stock solution diluted to 5 mL with methanol, *Crocus sativus L.* extract solution prepared with 2 mL of corresponding stock solution diluted to 5 mL with methanol, and the Olive fruit / Red Yeast Rice / *Crocus sativus L.* extracts composition was prepared by mixing 1, 2, and 2 mL of the corresponding stock solutions. The absorbance of the DPPH $10^{-4}$ M solution was measured at $t = 0$ ($A_0$), against methanol as blank. For each of the tested pure extracts/compositions, 50 $\mu$L of their methanolic solution was pipetted in a cuvette and subsequently 3.0 mL of DPPH $10^{-4}$ M solution were added and the solution was gently mixed. The absorbance ($A_T$) was measured after 60 min (the value reached a plateau after 40 min). For each measurement, a solution of the corresponding extract/combination in methanol was used as the blank (50 $\mu$L of sample methanolic solution mixed with 3.0 mL methanol). For each of the samples tested for its radical scavenging activity the % reduction in DPPH absorbance was estimated as ($A_0$ - $A_T$) * 100 / $A_0$.

The values in the Tables are expressed as the mean values of at least five determinations. All experiments were repeated in duplicate. The SD is estimated to be below 10% for all experiments.

**Table 1. Results of DPPH absorbance before and after the addition of Olive fruit /Red Yeast Rice Extracts Composition and its bioactive constituents at $\lambda_{max}$ = 515 nm.**

| Sample | Initial Conc. (mg sample /mL methanol) | Final Conc ($\mu$g sample /mL DPPH solution) | $A_0$ | $A_T$ | Reduction in DPPH Absorbance (%) |
|---|---|---|---|---|---|
| Olive fruit extract | 0.72 | 11.80 | 0.5404 | 0.2162 | 60.0 |
| Red Yeast Rice Extract | 4.64 | 76.07 | 0.5404 | 0.5222 | 3.4 |
| Olive fruit /Red Yeast Rice Extracts Composition | 5.36 | 87.87 | 0.5404 | 0.1607 | 70.3 |
| $A_0$, initial DPPH absorbance. $A_T$, final DPPH absorbance in the reaction medium (steady state). | | | | | |

**Table 2. Results of DPPH absorbance before and after the addition of Olive fruit / Red Yeast Rice / *Crocus sativus L.* Extracts Composition and its bioactive constituents at $\lambda_{max}$ = 630 nm.**

| Sample | Initial Conc. (mg sample /mL methanol) | Final Conc ($\mu$g sample /mL DPPH solution) | $A_0$ | $A_T$ | Reduction in DPPH Absorbance (%) |
|---|---|---|---|---|---|
| Olive fruit extract | 0.72 | 11.80 | 0.1894 | 0.0784 | 58.6 |
| Red Yeast Rice Extract | 4.64 | 76.07 | 0.1894 | 0.1813 | 4.3 |
| *Crocus sativus L.* Extract | 4.21 | 68.98 | 0.1894 | 0.1865 | 1.6 |
| Olive fruit /Red Yeast Rice/ *Crocus sativus L.* Extracts Composition | 9.57 | 156.85 | 0.1894 | 0.0648 | 65.8 |
| $A_0$, initial DPPH absorbance. $A_T$, final DPPH absorbance in the reaction medium (steady state). | | | | | |

Due to high absorbance of *Crocus sativus L.* extract at 515 nm, the DPPH antiradical scavenging activity test also performed at 630 nm, where DPPH also presents a linear calibration curve in the concentration range of 5 to 1000 mM.

In this context, Table 1 reveals the synergistic effect of the combination of Olive fruit and Red Yeast rice extracts as expressed by the % DPPH reduction of the combination of the two extracts compared to the sum of the % DPPH reduction of each one.

Most importantly, a representative embodiment of the invention with the combination of the three extracts reduces the absorbance of a $10^{-4}$ M DPPH solution in a greater degree compared to the corresponding reduction from each extract, furthermore, it presents a greater performance than the combined sum of the three individual extracts, thus, indicating that the unique combination of Olive fruit, Red Yeast Rice and *Crocus sativus L.* extracts act synergistically in the % absorbance reduction of DPPH and therefore present a synergistic antioxidant activity.

**EP 3 773 514 B1**

**Claims**

1. Soft capsules suitable for oral administration, comprising as active ingredients the combination of olive fruit extract standardized to at least 7% of hydroxytyrosol, Red Yeast Rice extract standardized to at least 4% of monacolin K and *Crocus sativus L*. extract standardized to at least 25% of crocin, wherein the combination is encapsulated in the soft capsule and homogeneously distributed in a hydrophobic matrix.

2. Soft capsule according to claim 1, wherein the hydrophobic matrix comprises an edible oil.

3. Soft capsule according to claim 1 or 2, wherein the edible oil is a vegetable oil selected from coconut oil, corn oil, cotton seed oil, olive oil, palm oil, peanut oil, rape seed oil, safflower oil, sesame oil, soy bean oil, and sunflower oil.

4. Soft capsule according to claim 1, wherein the vegetable oil is olive oil.

5. Soft capsule according to any of claims 1 to 4, comprising an edible olive oil, in a quantity which is in the range between 200 mg and 400 mg, more preferably, 240 mg per dosage unit.

6. Soft capsule according to any of claims 1 to 5, further comprising an emulsifier, most preferably lecithin, in a quantity which is in the range between 80 mg and 160 mg, more preferably, 100 mg per dosage unit.

7. Soft capsule according to any of claims 1 to 6, comprising a quantity of the olive fruit extract in a range of between 30 mg and 70 mg per dosage unit, preferably, 35 mg per dosage unit.

8. Soft capsule according to any of claims 1 to 7, comprising a quantity of the Red Yeast Rice extract in a range of between 50 mg and 200 mg per dosage unit, preferably, 125 mg per dosage unit.

9. Soft capsule according to any of claims 1 to 8, comprising a quantity of *Crocus sativus L.* extract in a range of between 70 mg and 200 mg per dosage unit, preferably, 100 mg per dosage unit.

10. Method for the preparation of soft capsules according to any one of claims 1-9, encapsulating a stable composition, comprising hydroxytyrosol, monacolin K and crocin as active ingredients, homogeneously distributed in a matrix, wherein said method comprises the steps of:

    a) sieving the solid active ingredients separately through a sieve for exclusion of particles, having a particle diameter of greater than 250 $\mu$m;
    b) mixing the first of the active ingredients with an edible oil, and optionally an emulsifier at a temperature in the range of 45 °C and 75 °C;
    c) dispersing the second of the active ingredients, optionally under agitation into the product obtained in step b) at a temperature in the range of 45 °C to 75 °C;
    d) cooling down of the product obtained in step c) to a temperature in the range of 25 °C and 35 °C, optionally followed by a homogenizing treatment, wherein the product is passed through a mill; and
    e) dispersing in the product obtained in step d) of the third active ingredient at a temperature in the range of 25 °C and 35 °C.

11. Method according to claim 10, wherein the first and/or the second active ingredient is an extract of olive fruit, or Red Yeast Rice and the third active ingredient is *Crocus sativus L.*.

12. Method according to claim 10 or 11, wherein the method comprises the steps of:

    a) sieving the extracts of olive fruit, of Red Yeast Rice and *Crocus sativus L.* as active ingredients through a 60 mesh sieve,
    b) mixing the sieved olive fruit extract, the edible olive oil and the emulsifier at a temperature between 45 °C and 75 °C,
    c) dispersing the sieved Red Yeast Rice extract in the oily mixture of step b) and stirring at the same temperature,
    d) cooling the mixture of step c) to a temperature between 25 °C and 35 °C, followed by homogenization,
    e) dispersing the sieved *Crocus sativus L.* extract into the homogeneous oily mixture of step d) and stirring at a temperature between 25 °C and 35 °C,
    f) cooling the mixture of step e) at 25 °C and homogenizing through a colloidal mill.

**Patentansprüche**

1. Weichkapseln, die zur oralen Verabreichung geeignet sind und als Wirkstoffe die Kombination von Olivenfruchtextrakt, der auf mindestens 7 % Hydroxytyrosol standardisiert ist, Rothefe-Reis-Extrakt, der auf mindestens 4 % Monacolin K standardisiert ist, und *Crocus sativus L.*-Extrakt, der auf mindestens 25 % Crocin standardisiert ist, enthalten, wobei die Kombination in der Weichkapsel eingekapselt und homogen in einer hydrophoben Matrix verteilt ist.

2. Weichkapsel nach Anspruch 1, wobei die hydrophobe Matrix ein Speiseöl umfasst.

3. Weichkapsel nach Anspruch 1 oder 2, wobei das Speiseöl ein pflanzliches Öl ist, ausgewählt aus Kokosnussöl, Maisöl, Baumwollsamenöl, Olivenöl, Palmöl, Erdnussöl, Rapsöl, Safloröl, Sesamöl, Sojabohnenöl und Sonnenblumenöl.

4. Weichkapsel nach Anspruch 1, wobei das Pflanzenöl Olivenöl ist.

5. Weichkapsel nach einem der Ansprüche 1 bis 4, enthaltend ein essbares Olivenöl in einer Menge im Bereich zwischen 200 mg und 400 mg, vorzugsweise 240 mg pro Dosierungseinheit.

6. Weichkapsel nach einem der Ansprüche 1 bis 5, die ferner einen Emulgator, vorzugsweise Lecithin, in einer Menge im Bereich zwischen 80 mg und 160 mg, vorzugsweise 100 mg, pro Dosierungseinheit enthält.

7. Weichkapsel nach einem der Ansprüche 1 bis 6, die eine Menge des Olivenfruchtextrakts in einem Bereich zwischen 30 mg und 70 mg pro Dosierungseinheit, vorzugsweise 35 mg pro Dosierungseinheit, enthält.

8. Weichkapsel nach einem der Ansprüche 1 bis 7, umfassend eine Menge des Rothefe-Reis-Extrakts in einem Bereich zwischen 50 mg und 200 mg pro Dosierungseinheit, vorzugsweise 125 mg pro Dosierungseinheit.

9. Weichkapsel nach einem der Ansprüche 1 bis 8, umfassend eine Menge an *Crocus sativus L.*-Extrakt in einem Bereich zwischen 70 mg und 200 mg pro Dosierungseinheit, vorzugsweise 100 mg pro Dosierungseinheit.

10. Verfahren zur Herstellung von Weichkapseln nach einem der Ansprüche 1-9, bei dem eine stabile Zusammensetzung eingekapselt wird, die Hydroxytyrosol, Monacolin K und Crocin als Wirkstoffe umfasst, die homogen in einer Matrix verteilt sind, wobei das Verfahren die folgenden Schritte umfasst:

    a) getrenntes Sieben der festen Wirkstoffe durch ein Sieb zum Ausschluss von Teilchen mit einem Teilchendurchmesser von mehr als 250 μm;
    b) Mischen des ersten Wirkstoffs mit einem Speiseöl und gegebenenfalls einem Emulgator bei einer Temperatur im Bereich von 45 °C und 75 °C;
    c) Dispergieren des zweiten Wirkstoffs, gegebenenfalls unter Rühren, in dem in Schritt b) erhaltenen Produkt bei einer Temperatur im Bereich von 45 °C bis 75 °C;
    d) Abkühlen des in Schritt c) erhaltenen Produkts auf eine Temperatur im Bereich von 25 °C und 35 °C, gegebenenfalls gefolgt von einer Homogenisierungsbehandlung, wobei das Produkt durch eine Mühle geleitet wird; und
    e) Dispergieren des dritten Wirkstoffs in dem in Schritt d) erhaltenen Produkt bei einer Temperatur im Bereich von 25 °C und 35 °C.

11. Verfahren nach Anspruch 10, wobei der erste und/oder der zweite Wirkstoff ein Extrakt aus Olivenfrüchten oder Red Yeast Rice ist und der dritte Wirkstoff *Crocus sativus L.* ist.

12. Verfahren nach Anspruch 10 oder 11, wobei das Verfahren die folgenden Schritte umfasst:

    a) Sieben der Extrakte aus Olivenfrüchten, Red Yeast Rice und *Crocus sativus L.* als Wirkstoffe durch ein 60-Mesh-Sieb,
    b) Mischen des gesiebten Olivenfruchtextrakts, des essbaren Olivenöls und des Emulgators bei einer Temperatur zwischen 45 °C und 75 °C,
    c) Dispergieren des gesiebten Red Yeast Rice-Extrakts in der öligen Mischung aus Schritt b) und Rühren bei gleicher Temperatur,

d) Abkühlen der Mischung aus Schritt c) auf eine Temperatur zwischen 25 °C und 35 °C und anschließendes Homogenisieren,

e) Dispergieren des gesiebten *Crocus sativus L.*-Extrakts in der homogenen öligen Mischung von Schritt d) und Rühren bei einer Temperatur zwischen 25 °C und 35 °C,

f) Abkühlen der Mischung aus Schritt e) auf 25 °C und Homogenisieren durch eine Kolloidmühle.

**Revendications**

1. Capsules molles appropriées pour une administration orale, comprenant comme principes actifs l'association d'extrait d'olives standardisé à au moins 7 % d'hydroxytyrosol, d'extrait de levure de riz rouge standardisé à au moins 4 % de monacoline K et d'extrait de *Crocus sativus L.* standardisé à au moins 25 % de crocine, l'association étant encapsulée dans la capsule molle et répartie de manière homogène dans une matrice hydrophobe.

2. Capsule molle selon la revendication 1, dans laquelle la matrice hydrophobe comprend une huile alimentaire.

3. Capsule molle selon la revendication 1 ou 2, dans laquelle l'huile alimentaire est une huile végétale choisie parmi l'huile de coprah, l'huile de maïs, l'huile de coton, l'huile d'olive, l'huile de palme, l'huile d'arachide, l'huile de colza, l'huile de carthame, l'huile de sésame, l'huile de soja et l'huile de tournesol.

4. Capsule molle selon la revendication 1, dans laquelle l'huile végétale est l'huile d'olive.

5. Capsule molle selon l'une quelconque des revendications 1 à 4, comprenant une huile d'olive alimentaire, en une quantité qui est dans la plage comprise entre 200 mg et 400 mg, plus préférablement, 240 mg par unité posologique.

6. Capsule molle selon l'une quelconque des revendications 1 à 5, comprenant en outre un émulsifiant, au mieux de la lécithine, en une quantité qui est dans la plage comprise entre 80 mg et 160 mg, plus préférablement, 100 mg par unité posologique.

7. Capsule molle selon l'une quelconque des revendications 1 à 6, comprenant une quantité de l'extrait d'olives dans une plage comprise entre 30 mg et 70 mg par unité posologique, de préférence, 35 mg par unité posologique.

8. Capsule molle selon l'une quelconque des revendications 1 à 7, comprenant une quantité de l'extrait de levure de riz rouge dans une plage comprise entre 50 mg et 200 mg par unité posologique, de préférence, 125 mg par unité posologique.

9. Capsule molle selon l'une quelconque des revendications 1 à 8, comprenant une quantité d'extrait de *Crocus sativus L.* dans une plage comprise entre 70 mg et 200 mg par unité posologique, de préférence, 100 mg par unité posologique.

10. Procédé de préparation de capsules molles selon l'une quelconque des revendications 1 à 9, encapsulant une composition stable, comprenant de l'hydroxytyrosol, de la monacoline K et de la crocine en tant que principes actifs, répartie de manière homogène dans une matrice, dans lequel ledit procédé comprend les étapes de :

    a) tamisage des principes actifs solides séparément à travers un tamis d'exclusion de particules, ayant un diamètre de particule supérieur à 250 μm ;
    b) mélange du premier des principes actifs avec une huile comestible, et éventuellement un émulsifiant à une température dans la plage de 45 °C à 75 °C ;
    c) dispersion du deuxième des principes actifs, éventuellement sous agitation dans le produit obtenu à l'étape b) à une température dans la plage de 45 °C à 75 °C ;
    d) refroidissement du produit obtenu à l'étape c) à une température dans la plage de 25 °C à 35 °C, éventuellement suivi d'un traitement d'homogénéisation, dans lequel le produit est passé à travers un broyeur ; et
    e) dispersion dans le produit obtenu à l'étape d) du troisième principe actif à une température dans la plage de 25 °C à 35 °C.

11. Procédé selon la revendication 10, dans lequel le premier et/ou le deuxième principe actif est un extrait d'olives, ou de levure de riz rouge et le troisième principe actif est *Crocus sativus L.*

**12.** Procédé selon la revendication 10 ou 11, dans lequel le procédé comprend les étapes de :

a) tamisage des extraits d'olives, de levure de riz rouge et de *Crocus sativus L.* en tant que principes actifs à travers un tamis de 60 mesh,

b) mélange de l'extrait d'olives tamisé, de l'huile d'olive comestible et de l'émulsifiant à une température comprise entre 45 °C et 75 °C,

c) dispersion de l'extrait de levure de riz rouge tamisé dans le mélange huileux de l'étape b) et agitation à la même température,

d) refroidissement du mélange de l'étape c) à une température comprise entre 25 °C et 35 °C, suivi d'une homogénéisation,

e) dispersion de l'extrait de *Crocus sativus L.* tamisé dans le mélange huileux homogène de l'étape d) et agitation à une température comprise entre 25 °C et 35 °C,

f) refroidissement du mélange de l'étape e) à 25 °C et homogénéisation à travers un broyeur colloïdal.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2170090 A
- WO 2010069951 A
- WO 2012114133 A
- WO 2009013596 A2

**Non-patent literature cited in the description**

- **BRAND-WILLIAMS, W. et al.** *Lebensm.-Wiss. u.-Technol.,* 1995, vol. 28, 25-30